# EUROPEAN PATENT SPECIFICATION

(11) **EP 0 463 086 B1**
(45) Date of publication and mention of the grant of the patent: **11.05.1994**
(21) Application number: 90905392.8
(22) Date of filing: 20.03.1990
(51) Int. Cl.: A61M 5/32

(54) **SYRINGE WITH INTERCHANGEABLE AND RETRACTABLE NEEDLE PLATFORM**
SPRITZE MIT AUSWECHSELBARER UND ZURÜCKZIEHBARER KANÜLENPLATTFORM
SERINGUE AVEC PLATE-FORME D'AIGUILLE INTERCHANGEABLE ET RETRACTIBLE

(30) Priority: 22.03.1989 US 327344; 21.09.1989 US 410318
(43) Date of publication of application: 02.01.1992
(73) Proprietor: INVIRO MEDICAL DEVICES LTD., Bridgetown (BB)
(72) Inventor: Novacek, Laurel A., Vancouver, British Columbia V6S 1G6 (CA); Sharp, Fraser Rosslyn, Vancouver, British Columbia V6J 1C5 (CA); McLean, Donald A., Vancouver, British Columbia V6R 1V3 (CA)
(74) Representative: Raynor, John
(86) International application number: PCT/CA90/00095
(87) International publication number: WO 90/11099

(56) References cited:
- EP-A- 0 278 493
- EP-A- 0 347 742
- WO-A-89/12475
- GB-A- 1 150 980
- US-A- 4 675 005
- US-A- 4 747 830
- US-A- 4 790 828
- US-A- 4 813 936
- US-A- 4 861 338

## Description

This invention relates to a novel safety disposal syringe needle device which has chemical and industrial application. More particularly, this invention pertains to a syringe which, after being used by a person to inject medication or fluid into a patient, or withdraw fluids from a patient after sampling or exposure to toxic materials, or the like, can be transformed by the person to withdraw the needle into the barrel of the syringe for disposal purposes, thereby eliminating needlestick injuries among such persons.

A number of patents disclose syringes or the like having needle protecting features. Examples of such patents are US-A-4592744, US-A-4804370, US-A-4542749, US-A-3306290, US-A-4631057, US-A-4425120, US-A-4573976, US-A-3884230, US-A-4258713, US-A-4085737, US-A-4266543, US-A-4266544, and US-A-4774964.

US-A-4747830 discloses a syringe with a plunger having an enlarged head, which passes through an opening in an adaptor assembly to engage a shoulder, such that the adaptor and needle can be withdrawn from the syringe barrel.

US-A-4861338 concerns a syringe which requires a member which mounts the needle to be screwed back into the barrel upon rotary action of the plunger. No means are provided for grasping the adaptor, to withdraw the adaptor.

EP-A-347742 relates to a syringe with a threaded connection between its plunger, and a member carrying the needle.

US-A-4675007 again concerns a simple arrangement with a threaded connection between a needle-carrying member, and a plunger.

WO-A-89/12475 is also concerned with a syringe which permits the use of standard hypodermic needles which may be retracted into the body of the syringe after use. A needle carrier or adaptor is removably engaged in the body of the syringe, and includes splines which engage with corresponding splines on the plunger. Attachment between the plunger and the adaptor relies however on a frictional engagement, and there is no latching mechanism disclosed to provide positive engagement between the plunger and the adaptor.

In accordance with the present invention, there is provided a syringe comprising:-
(a) a hollow axially elongate barrel (8),
(b) An adaptor (102) for carrying a hollow needle (2), the adaptor being removably mounted at the distal end of the barrel (8) and having a central fluid passageway,
(c) a plunger (16) axially moveable in the hollow barrel (8), and
(d) latching means (106,108) associated with the distal end of the plunger (16) for engaging the adaptor (102) and means for causing the adaptor (102) to rotate so as to cause the adaptor to part from the distal end of the barrel, and allow the adaptor (102) and a needle carried by the adaptor to be withdrawn into the barrel (8) on withdrawal of the plunger from an advanced position to a retracted position,
characterised in that driving faces (150) are provided associated with the plunger (16) adapted to mate with corresponding driving faces (151) associated with the adaptor (102) for effecting a driving connection between the plunger (16) and the adaptor (102) so as to cause rotation of the adaptor (102) relative to the barrel (8) on rotation of the plunger (16) within the barrel (8) thereby to cause the adaptor to part from the distal end of the barrel, and
in that camming surfaces (118) are provided associated with the adaptor (102) for causing relative rotation of the plunger (16) and the adaptor (102) in response to axial movement of the plunger (16) in the barrel (8) in such a manner as to cause engagement of the said drive surfaces (104) on the said axial movement and consequent relative rotation of the plunger (16) and barrel (8).

After being used by a health care person to inject medication or fluid into a patient or withdraw fluids from a patient, the syringe in accordance with the invention can be transformed by that person to withdraw the needle into the barrel of the syringe for disposal purposes, thereby eliminating the occurrence of needle sticking injuries among such health care persons. This syringe can also be used in industrial processes for sampling or adding substances which may be toxic. After such a function, the needle is withdrawn into the barrel to prevent contamination at any further point in the process or during disposal. The syringe and retractable needle feature is adapted to be used with a variety of interchangeable needles of different diameter and length utilizing a universal Luer lock coupling mechanism.

The needle may be engaged by a female and male thread combination by rotating the plunger relative to the barrel and needle. Alternatively, the needle may be engaged by a com-lock combination.

The piston engaging means of the adaptor can be a self-aligning, non-jamming spiral flight - flat driving surface combination and the releasable engagement means on the piston can be flat driving faces which engage with the flat driving faces of the adaptor. The adaptor can be releasably connected to the end of the barrel means by a female-male thread combination, and the adaptor may comprise a spiral flight-flat-driving face combination adapted to engage a flat driving face engaging means of the plunger.

The needle may be fitted with a Luer lock, the Luer lock being engaged with the adaptor. The needle-Luer lock combination and the adaptor can be disengaged from the end of the barrel means by latch means which engages with the adaptor when the plunger is pushed to the needle end of the barrel and rotated.

The adaptor can protrude partially from the distal end of the barrel and can have a thread direction which is the same as or opposite to the thread direction of the barrel engaging the needle. The adaptor can be designed to protrude partially from the distal end of a syringe barrel.

A number of preferred embodiments of the invention are in the accompanying drawings in which:
Figure 1 illustrates a side elevation partial section view of an embodiment of the syringe wherein the piston is adapted with a latch which snaps into place in an adapter after the piston is fully depressed and rotated.
Figure 2 illustrates a side elevation partial section view of the adaptor shown in Figure 1;
Figure 3 illustrates a side elevation section view taken along section line C-C of Figure 4;
Figure 4 illustrates an end view of the latch mechanism of the piston depicted in Figure 1;
Figure 5 illustrates a side elevation view of an alternative design of adaptor;
Figure 6 illustrates a side elevation partial section view of the adaptor of Figure 5; and
Figure 7 illustrates a side elevation partial section view of an embodiment of the syringe wherein an adaptor is mounted so that it partially extends from the front end of the barrel.

Most disposable syringes can be used with a variety of interchangeable needles with different diameter and length, The needles are connected by what is known as a Luer connector, which may be of two types. One is a simple conical device which accepts the needle base. This version is often described as a Luer lock. The Luer lock has a simple screw thread locking mechanism that permits the base of the needle to be screwed onto the syringe so that it cannot be pulled off without unscrewing. In this disclosure, the universal coupling mechanism connecting the needle to the syringe will be referred to as a Luer lock version of the Luer connector unless otherwise indicated. It should be recognised that the claims to the invention relate to both the plain Luer tip and the Luer lock mechanisms. The interchangeability capability of a Luer lock allows for the most appropriate needle to be used for syringe filling and patient injection. In many cases, to save time, a different larger needle is used to fill the syringe with fluid prior to injection. A needle of fine calibre to minimize pain to the patient, and tissue damage, is often used for intra-muscular of subcutaneous injection. In addition, if the same needle is used to puncture a vial in order to fill the syringe with medication, there is a potential for contamination of that needle from the vial, if the vial stopper carries a contaminant. Under most circumstances, this would not pose a significant risk. However, if the patient has reduced immunity to infection, the ability to change to an entirely new sterile needle for patient injection may become important.

Although the prior art describes syringes which can protect the needle by a variety of means, including those which involve withdrawing the needle into the interior of the barrel of the syringe, such syringes do not allow for interchangeability of the needle or for the universal Liner lock coupling mechanism which is an important feature of the syringe. Most commercially available syringes employ a Luer lock. Since the preferred embodiment of the present invention is adapted for use with a Luer lock, it can directly replace syringes currently in use and requires no change in technique or procedure until after the syringe has been used. In addition, most currently produced needles can be used in the manner on this syringe.

After use, once the syringe has been withdrawn into the barrel, the syringe plunger can in the preferred embodiment, be snapped off. It is designed so that it can be screwed onto the front of the syringe and thereby prevent any possibility of the needle within the barrel protruding through the front of the syringe again. This is an important factor for a health care worker using the syringe and also for any health care workers subsequently handling garbage which might contain a contaminated syringe.

This invention pertains to a syringe which, after being used by a health care worker or a hazardous industries worker, or the like, to inject medication or fluid into a patient, or withdraw fluid from a patient, or in sampling toxic material, for example, in an industrial process, can be transformed by the worker to withdraw the needle into the barrel of the syringe for disposal purposes, thereby eliminating potentially harmful needlestick injuries among such workers. In industrial applications, the storage of a contaminated needle is similarly effected within the barrel to prevent further contamination of the environment or process.

The needle is retracted by the user into the interior of the body of the syringe immediately after it is withdrawn from the patient's body tissue, or after exposure to hazardous situations. Thus, the needle is not exposed for accidental contact at any time after the needle has contacted the potentially hazardous patient's body fluids, or other hazardous materials. The retraction feature eliminates the possibility of potentially dangerous needlestick injuries occurring with contaminated needles.

The safety syringe of the invention is simple to operate and is only slightly more expensive to manufacture than presently used syringes. Another advantage is that the syringe design closely resembles currently used syringes and thus there should be no difficulty in obtaining good acceptance among workers such as medical institutional workers. Moreover, the operation of the subject syringe is easy to teach to such workers and requires no unusual skills or manual dexterity.

Syringes that are in current commercial use normally consist of four components, a needle cap which is removed prior to use, a hollow needle which is mounted on a hub with a Luer lock, a barrel to which the hub is attached, and a plunger with a bung (piston) at the head end of the plunger. The plunger is inserted within the barrel head end first and can be pushed into the interior of the barrel in order to pump fluid contained in the barrel out through the interior of the hollow needle. The subject invention, in various embodiments, includes several basic modifications which do not dramatically change the appearance of the conventional syringe.

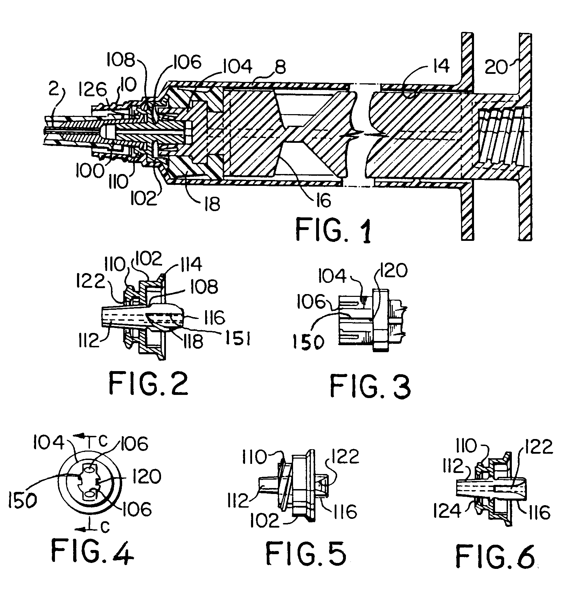

Figure 1, illustrates a side elevation partial-section view of an embodiment of the syringe wherein the piston is adapted with a latch which snaps into place in an adaptor (platform) after the piston is fully depressed and rotated clockwise. The embodiment illustrated in Figure 1 depicts the needle 2 embedded in a Luer lock 100, associated with the constricted end 10 of syringe barrel 8. The plunger 16 with a finger press 20 at the remote end thereof is positioned inside barrel 8. A stop catch 14 prevents the plunger 16 from being totally withdrawn from the interior of the barrel 8. Bung 18, which provides a tight fit with the interior of the barrel 8, is mounted on the end of plunger 16 opposite finger press 20.

The operative needle engagement and detachment mechanism illustrated in Figure 1 is a combination of an adapter 102, which cooperates with Luer lock 100, the combination fitting into the narrow end 10 of barrel 8. The end of plunger 16 opposite finger press 20 has a latch mechanism 104 formed inside bung 18. A pair of prongs 106 are formed in latch 104 and engage into grooves 108 in adapter 102 when the plunger 16 is fully advanced. The plunger 16 - adapter 102 engagement mechanism and subsequent adapter 102 - needle 2 disengagement mechanism has sequential aspects as follows: 1) initial full depression of the piston and plunger 16 which causes automatic self alignment of the latch 104 and of the flat driving faces 150 of ridges 120 with the flat driving faces 151 of the adapter because of the self-aligning non-jamming spiral threads 118, 2) rotation of the plunger 16 which causes unseating of the adapter 102 at threads 110, 3) latching of the prongs 106 in grooves 108, and 4) withdrawal of the adapter 102 with the attached needle 2 into the barrel 8 of the syringe by withdrawing the plunger 16 by finger press 20. The pair of prongs 106 engaging in the respective grooves 108 of the adapter 102, enable the disengaged adapter 102, and the needle with the Luer lock 100, to be withdrawn. At this point, the needle 2, Luer lock 100, adapter 102, and bung 18 can be withdrawn into the interior of the barrel 8 by pulling finger press 20 away from the narrow end 10 of the syringe.

As can be recognized, the adapter 102 is an important feature of this embodiment of the invention. The adapter enables a standard Luer lock 100 to indirectly mate with latch 104 at the end of plunger 16. Moreover, the adapter 110 is designed so that it accommodates different sizes of needle 2 and Luer lock 100. Moreover, the adapter is designed so that it accommodates different sizes of needles via a Luer lock plain tip or other mechanism depending on the configuration of the outer coupling adapter.

Figure 1 illustrates a narrow point in the plunger which assists in breaking the plunger in two. If required, or desirable, two or more additional narrow points can be included to permit breakage at alternative locations.

Figure 2 illustrates in side elevation partial section view a preferred embodiment of the adapter 102. Nose 112 is adapted to fit inside the hollow of a standard Luer lock needle base or needle 100. The left hand thread 110 is also shown in Figure 27. The adapter 102 has opposite the nose 112 a cup-like edge 114 which is formed to receive the front end of latch 104. Formed inside the rim of cup 114 is a protrusion 116 which has a pair of self-aligning, non-jamming spiral male threads 118 formed thereon. A pair of flat driving faces 150 are also formed in protrusion 116. Prong engaging grooves 108 are also formed in the interior of the protrusion 116 at the point where the protrusion 116 joins with the cup 114. The advantage of this adapter design is that with the fast acting spiral threads 118 force alignment without jamming. Full axial movement of the plunger is possible with no rotation of the adapter forced by the alignment grooves because of the torque inflexion inherent in the plunger. Then after full depression of the plunger, and engagement of the flat driving faces 150 with faces 151, deliberate rotation increases the flexion to its limit. Further rotation causes the adapter 102 to rotate and disengage the thread seating in the front of the barrel 8. During this procedure, the prongs 106 and grooves 108 engage as the adapter is unseated, allowing the entire internal assembly to be withdrawn. At that point, further clockwise rotation of the plunger 16 by means of finger press 20 causes the threads 110 of adapter 102 to disengage from the interior narrow end 10. After unseating, the prone groove attachment can, on its own, allow the needle 2 assembly with prongs 106 engaged in grooves 108 to be withdrawn into the interior barrel 8 simply by withdrawing finger press 20.

Figure 3 illustrates a section view taken along section lines C-C of Figure 29. Figure 3 depicts a detailed view of the construction of the latch 104 and prongs 106. In the embodiment illustrated in Figure 3, the latch 104 has a pair of alignment ridges 120 formed in the interior of latch 104. These alignment ridges 120 assist engagement of the latch 104 with adapter 102.

Figure 5, which illustrates a side view of an alternative design of adapter 102, and Figure 6, which illustrates a partial cutaway section view of the adapter depicted in Figure 5, illustrate flared grooves 122, which are adapted to receive ridges 120 of latch 104. The flare assists in enabling the ridges 120 to be received into grooves 122. In this embodiment, the adapter does not have the fast acting non-jamming spiral threads 118, depicted in the adapter design illustrated in Figure 2.

The adapter 102 has a rim 124 which is designed to engage with the rim 126 of the Luer lock 100 to hold the two snugly together, and provide a fluid seal by the complementary configuration of the adapter and the front end of the syringe.

The embodiments of the invention depicted in Figures 1 to 6 have a number of advantages:
1. The preferred embodiment allows for universal coupling with all Luer lock needle connections.
2. Needle interchangeability during use of the syringe is possible, that is, different needles can be used for filling the vial and for injecting the patient.
3. The needle platform (adapter) design allows for compatibility of the syringe with other custom design needles or any subsequent needle design, merely by altering the outer needle connection configuration platform.
4. The syringe hub can be permanently closed after use of the syringe by screwing on the broken plunger stalk after withdrawal of the needle into the barrel.
5. The novel coupling mechanism between the platform and the plunger allows for full axial movement of the plunger. Although transient mating of the prongs and grooves may occur, no functional attachment occurs until the adapter is unseated from the front of the barrel. At that time, the prong and groove attachment becomes functional to allow withdrawal of the platform (adapter) with the attached needle into the barrel.

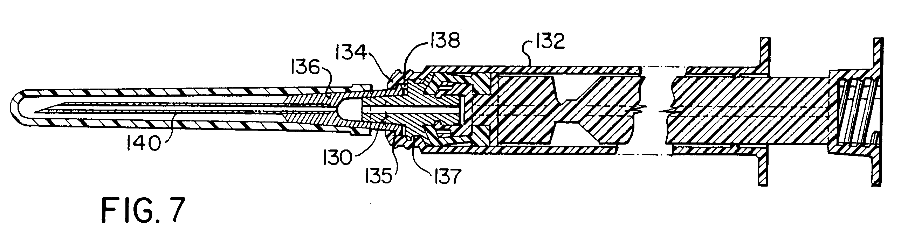

Figure 7 illustrates a side elevation partial side section view of an embodiment of the invention related to that illustrated in Figures 1 to 5. The embodiment depicted in Figure 7 shows an adapter 130 which extends partially from the front end of the barrel 132. A standard Luer lock 134 is formed in the front end of the barrel 132. The Luer lock has a standard right hand thread 135. The barrel 132 has a left hand thread 137 to release the adapter 130 for withdrawal inside the barrel 132, after the syringe is used. A needle base 136 carrying needle 140 fits on the tapered front end of adapter 130 and screws into the right hand thread of the Luer lock 134. A given clearance 138 permits the needle base 136 to be withdrawn into the interior of the barrel 132 after the adapter 130, base 136, and needle 140 are unscrewed and withdrawn into the barrel 132 by rotation of the plunger and engagement of the plunger adapter connection mechanism. As described previously, a Luer tip may be a simple conical device which accepts the needle base. This design could also be used in this embodiment, thus eliminating the screw connection of the needle base 136 and the Luer lock thread 135.

Another advantage is that by reducing the diameter of the platform needle combination, a thinner barrel can be manufactured. For a given capacity of syringe, this will allow the length of the syringe to be longer and therefore a longer needle to be accommodated within it. By placing the Luer lock threads in the barrel of the syringe rather than on the platform, the outer diameter of the platform needle combination is defined by the width of the needle base and not by the supporting outer plastic Luer lock mechanism. It is therefore possible to ensure as long a barrel as possible for a given capacity. The standard 3 cc syringe represents a main portion of the syringe market. A standard 3 cc syringe is often used with a 38 mm (1-1/2 inch) needle for intramuscular injection. Accordingly, the barrel of a 3 cc syringe should be at least 64 mm (2-1/2 inches) long to accommodate the withdrawn needle, adapter and plunger connector all within the barrel.

The foregoing embodiments discuss various means of enabling the plunger to be connected securely to the base 6 of the needle 2, to enable the plunger 16, when withdrawn, to pull the hub 6 and needle 2 into the interior of the barrel 8. In some versions including the adapter, the plunger-adapter coupling transfers no rotary action if the plunger connection is twisted in the wrong direction. This provides an overtightening safety feature. The adapter is released from the syringe if the correct rotation is used. It will be recognized that these are illustrative of specific embodiments and there are other possible ways to make a secure connection for the purpose of withdrawing the adapter and needle into the interior of the barrel.

As will be apparent to those skilled in the art in the light of the foregoing disclosure, many alterations and modifications are possible in the practice of this invention without departing from the spirit or scope thereof. Accordingly, the scope of the invention is to be construed in accordance with the substance defined by the following claims.

## Claims

1. A syringe comprising:-
(a) a hollow axially elongate barrel (8),
(b) An adaptor (102) for carrying a hollow needle (2), the adaptor being removably mounted at the distal end of the barrel (8) and having a central fluid passageway,
(c) a plunger (16) axially moveable in the hollow barrel (8), and
(d) latching means (106,108) associated with the distal end of the plunger (16) for engaging the adaptor (102) and means for causing the adaptor (102) to rotate so as to cause the adaptor to part from the distal end of the barrel, and allow the adaptor (102) and a needle carried by the adaptor to be withdrawn into the barrel (8) on withdrawal of the plunger from an advanced position to a retracted position,
characterised in that
driving faces (150) are provided associated with the plunger (16) adapted to mate with corresponding driving faces (151) associated with the adaptor (102) for effecting a driving connection between the plunger (16) and the adaptor (102) so as to cause rotation of the adaptor (102) relative to the barrel (8) on rotation of the plunger (16) within the barrel (8) thereby to cause the adaptor to part from the distal end of the barrel, and
in that camming surfaces (118) are provided associated with the adaptor (102) for causing relative rotation of the plunger (16) and the adaptor (102) in response to axial movement of the plunger (16) in the barrel (8) in such a manner as to cause engagement of the said drive surfaces on the said axial movement and consequent relative rotation of the plunger (16) and barrel (8).

2. A syringe as claimed in Claim 1, wherein the adaptor (102) comprises a ferrule (100) for removably coupling the needle (2) to the adaptor (102).

3. A syringe as claimed in Claim 2, wherein the ferrule (100) and the adaptor (102) each comprise a threaded portion for the connection thereof to each other.

4. A syringe as claimed in any one of the preceding claims, wherein the adaptor (102) is detachably connected to the distal end of the barrel (8) by a threaded connection of a first sense and wherein said camming surfaces (118) include a threaded connection of a second sense, opposite to the said first sense.

5. A syringe as claimed in any one of the preceding claims, including means for causing the needle to releasably engage with needle coupling means on the adaptor, in response to rotation of the plunger end.

6. A syringe as claimed in any one of the preceding claims, wherein the adaptor (102) is releasably connected to the end of the barrel (8) by a female-male thread combination (110), the adapter (102) having a spiral thread and drive surfaces, the plunger end having driving surfaces for engaging the drive surfaces on the adapter (102) in response to relative rotation of the plunger end (16) and the adapter (102) and along said thread to align and engage the driving and drive surfaces of the plunger end and adapter (102) respectively, and thereafter for rotating the adapter (102) relative to the barrel (8) to enable axial withdrawal of the adapter (102) and needle ferrule along the barrel.

7. A syringe as claimed in any one of the preceding claims, wherein the needle includes a female Luer lock fitting, which is engaged with the adapter (102) with a plain Luer tip or Luer lock male fitting.

8. A syringe as claimed in any one of the preceding claims, wherein the adapter (102) is detachably connected to the distal end of the barrel (8) by a threaded connection of a first sense while the engagement structures between the adapter (102) and plunger (16) include a threaded connection of the same sense as said first sense.

9. A syringe as claimed in any one of the preceding claims, and wherein the said camming surfaces include a hollow central protrusion (104) on the plunger (16) having at least one helical track (118) around an outer surface of the protrusion.

10. A syringe as claimed in Claim 9, wherein the latch includes at least one snap-engagement (106) also disposed on the outer surface of said protrusion but circumferentially spaced from said at least one helical track.

11. A syringe as claimed in Claim 10, including a pair of said snap engagements (106), the members of each said pair being spaced apart approximately 180° circumferentially.

12. A syringe accordingly to any one of the preceding Claims, wherein the said latching means comprises a groove on one of said adapter (102) and said plunger (16) and a projection on the other of said adapter (102) and said plunger (16) for reception in said groove.

13. A syringe as claimed in any one of the preceding Claims, comprising a radially axially extending flat on each of said adapter (102) and said plunger (16) adapted to act as drive surfaces during said rotation of the plunger.

14. A syringe according to Claim 17, wherein said drive surfaces have an axial extent greater than their radial extent.

15. A syringe according to any one of the preceding claims, including a ferrule for carrying said needle, said ferrule having a male thread, said adapter (102) including a female threaded portion.

16. A syringe according to any one of the preceding claims, including male screw threads carried by the distal end of the barrel (8).

17. A syringe according to Claim 16, including female threads carried by the proximal end of the plunger (16), said plunger (16) having a weakened section intermediate its ends for breaking off a portion of the plunger (16), including the proximal end, upon withdrawal of the plunger (16) from the distal end of the barrel (8) whereby the broken-off portion may be threaded to the barrel (8) by causing the male and female threads on the barrel (8) and plunger end, respectively to cooperate.

18. A syringe according to any one of the preceding claims, including means on the barrel (8) and plunger (16) adapted to prevent total withdrawal of the plunger (16) from the barrel (8).

19. A syringe as claimed in any one of the preceding claims, wherein the latching means is such as to allow the plunger and adaptor to be released if desired.

## Patentansprüche

1. Spritze mit:
a) einem hohlen, in Axialrichtung länglichen Gehäuse (8),
b) einem Adapter (102) zum Tragen einer Hohlnadel (2), wobei der Adapter entfernbar an dem distalen Ende des Gehäuses (8) montiert ist und eine zentrale Durchführung für Flüssigkeit hat,
c) einem axial in dem hohlen Gehäuse (8) bewegbaren Kolben (16) und
d) einer mit dem distalen Ende des Kolbens (16) assoziierten Verriegelungseinrichtung (106, 108) zum Angreifen an dem Adapter (102) und Einrichtungen zum Bewirken, daß der Adapter (102) sich dreht, um so zu bewirken, daß sich der Adapter vom distalen Ende des Gehäuses trennt, und daß es ermöglicht wird, daß der Adapter (102) und eine von dem Adapter getragene Nadel beim Zurückziehen des Kolbens von einer vorgeschobenen Position in eine zurückgezogene Position in das Gehäuse (8) zurückgezogen wird,
**dadurch gekennzeichnet**, daß mit dem Kolben (16) assoziierte Antriebsflächen (150) vorgesehen sind, die so gestaltet sind, daß sie mit entsprechenden mit dem Adapter (102) assoziierten Antriebsflächen (151) zusammenpassen, um eine Antriebsverbindung zwischen dem Kolben (16) und dem Adapter (102) zu bewirken, um so bei einer Drehbewegung des Kolbens (16) in dem Gehäuse (8) eine Drehbewegung des Adapters (102) relativ zu dem Gehäuse (8) zu bewirken, wodurch bewirkt wird, daß sich der Adapter von dem distalen Ende des Gehäuses trennt, und
daß mit dem Adapter (102) assoziierte Kurvenflächen (118) vorgesehen sind, um als Folge einer axialen Bewegung des Kolbens (16) in dem Gehäuse (8) eine relative Drehbewegung des Kolbens (16) und des Adapters (102) auf eine solche Weise zu bewirken, daß bei der axialen Bewegung ein Eingriff der Antriebsflächen und eine folgende relative Drehbewegung des Kolbens (16) und des Gehäuses (8) bewirkt wird.

2. Spritze nach Anspruch 1, wobei der Adapter (102) eine Muffe (100) zum lösbaren Kuppeln der Nadel (2) mit dem Adapter (102) aufweist.

3. Spritze nach Anspruch 2, wobei die Muffe (100) und der Adapter (102) jeweils einen mit einem Gewinde versehenen Bereich für die Verbindung untereinander aufweisen.

4. Spritze nach einem der vorhergehenden Ansprüche, wobei der Adapter (102) mit dem distalen Ende des Gehäuses (8) durch eine Schraubverbindung mit einem ersten Schraubensinn abnehmbar verbunden ist und wobei die Kurvenflächen (118) eine Schraubverbindung mit einem zweiten Schraubensinn aufweisen, der dem ersten Schraubensinn entgegengesetzt ist.

5. Spritze nach einem der vorhergehenden Ansprüche, einschließlich einer Einrichtung zum Bewirken, daß die Nadel als Folge auf eine Drehbewegung des Kolbenendes lösbar an einer Nadelkupplungseinrichtung an dem Adapter eingreift.

6. Spritze nach einem der vorhergehenden Ansprüche, wobei der Adapter (102) mit dem Ende des Gehäuses (8) durch eine Innengewinde-Außengewinde-Kombination (110) lösbar verbunden ist, wobei der Adapter (102) ein Spiralgewinde und Antriebsflächen hat, wobei das Kolbenende antreibende Oberflächen zum Angreifen an den Antriebsflächen an dem Adapter (102) als Folge einer relativen Drehbewegung des Kolbenendes (16) und des Adapters (102) und entlang dem Gewinde hat, um die antreibende Oberfläche und die Antriebsfläche des Kolbenendes bzw. des Adapters (102) auszurichten und in Eingriff zu bringen, und zum Drehen des Adapters (102) relativ zu dem Gehäuse (8) danach, um einen axialen Rückzug des Adapters (102) und der Nadelmuffe entlang dem Gehäuse zu ermöglichen.

7. Spritze nach einem der vorhergehenden Ansprüche, wobei die Nadel ein weibliches Luer-Verriegelungsanschlußstück enthält, das an dem Adapter (102) mit einer einfachen Luer-Spitze oder einem männlichen Luer-Verriegelungsanschlußstück angreift.

8. Spritze nach einem der vorhergehenden Ansprüche, wobei der Adapter (102) durch eine Schraubverbindung mit einem ersten Schraubensinn abnehmbar mit dem distalen Ende des Gehäuses (8) verbunden ist, während die Angriffsstrukturen zwischen dem Adapter (102) und dem Kolben (16) eine Schraubverbindung mit demselben Schraubensinn wie dem ersten Schraubensinn aufweisen.

9. Spritze nach einem der vorhergehenden Ansprüche, wobei die Kurvenflächen einen hohlen zentralen Vorsprung (104) an dem Kolben (16) mit wenigstens einer schraubenförmigen Spur (118) um eine äußere Oberfläche des Vorsprungs aufweisen.

10. Spritze nach Anspruch 9, wobei die Verriegelung wenigstens ein Schnappeingriffselement (106) aufweist, das ebenso an der äußeren Oberfläche des Vorsprungs angeordnet ist, aber sich in Umfangsrichtung auf Abstand von der wenigstens einen schraubenförmigen Spur befindet.

11. Spritze nach Anspruch 10, einschließlich eines Paares der Schnappeingriffselemente (106), wobei die Teile des Paars in Umfangsrichtung sich auf einem Abstand von etwa 180° befinden.

12. Spritze gemäß einem der vorhergehenden Ansprüche, wobei die Verriegelungseinrichtung eine Aussparung an entweder dem Adapter (102) oder dem Kolben (16) und einen Vorsprung an dem jeweils anderen der beiden Teile Adapter (102) und Kolben (16) zur Aufnahme in der Aussparung aufweist.

13. Spritze nach einem der vorhergehenden Ansprüche, mit einem sich radial axial erstreckenden Flachteil an jeweils dem Adapter (102) und dem Kolben (16), das dazu angepaßt ist, als Antriebsflächen während der Drehbewegung des Kolbens zu wirken.

14. Spritze nach Anspruch 17, wobei die Antriebsflächen eine axiale Ausdehnung haben, die größer ist als ihre radiale Ausdehnung.

15. Spritze nach einem der vorhergehenden Ansprüche, einschließlich einer Muffe zum Tragen der Nadel, wobei die Muffe ein Außengewinde hat, wobei der Adapter (102) einen Bereich mit einem Innengewinde aufweist.

16. Spritze nach einem der vorhergehenden Ansprüche, einschließlich von dem distalen Ende des Gehäuses (8) getragenen Schraubenaußengewinden.

17. Spritze nach Anspruch 16, einschließlich von dem proximalen Ende des Kolbens (16) getragenen Innengewinden, wobei der Kolben (16) zwischen seinen Enden einen geschwächten Abschnitt zum Abbrechen eines Teils des Kolbens (16), einschließlich des proximalen Endes, beim Zurückziehen des Kolbens (16) vom distalen Ende des Gehäuses (8) hat, wodurch der abgebrochene Teil an das Gehäuse (8) geschraubt werden kann, indem bewirkt wird, daß die Außen- und Innengewinde an dem Gehäuse (8) bzw. dem Kolbenende zusammenwirken.

18. Spritze nach einem der vorhergehenden Ansprüche, einschließlich Einrichtungen an dem Gehäuse (8) und dem Kolben (16), die dazu geeignet sind, das vollständige Zurückziehen des Kolbens (16) von dem Gehäuse (8) zu verhindern.

19. Spritze nach einem der vorhergehenden Ansprüche, wobei die Verriegelungseinrichtung derart ist, daß sie erlaubt, daß der Kolben und der Adapter freigegeben werden, falls erwünscht.

## Revendications

1. Seringue comprenant
(a) un cylindre creux (8) allongé en direction axiale,
(b) un adaptateur (102) pour le montage d'une aiguille creuse (2), l'adaptateur étant amovible à l'extrémité distale du cylindre (8) et présentant un canal central pour le liquide,
(c) un plongeur (16) mobile axialement dans le cylinsdre creux (8), et
(d) des moyens de verrouillage (106, 108) associés à l'extrémité distale du plongeur (16) pour mettre en prise l'adaptateur (102), ainsi que des moyens pour faire tourner l'adaptateur (102) de sorte qu'il se détache de l'extrémité distale du cylindre et pour permettre à l'adaptateur (102) et à l'aiguille qu'il porte d'être abandonnés dans le cylindre (8) par un retrait du plongeur passant d'une position enfoncée à une position sortie,
caractérisée en ce qu'il est prévu des faces d'entraînement (150) associées au plongeur (16) agencé de façon à s'adapter sur des faces d'entraînement correspondantes (151) associées à l'adaptateur (102), pour établir une liaison d'entraînement entre le plongeur (16) et l'adaptateur (102), de façon à provoquer une rotation de l'adaptateur (102) par rapport au cylindre (8) lors de la rotation du plongeur (16) à l'intérieur du cylindre (8), amenant ainsi l'adaptateur à se détacher de l'extrémité distale du cylindre, et
en ce qu'il est prévu des surfaces de came (118) associées à l'adaptateur (102) pour provoquer une rotation relative au plongeur (16) et à l'adaptateur (102) en réponse à un mouvement axial du plongeur (16) dans le cylindre (8), de telle sorte que lesdites faces d'entraînement sont amenées à être mises en prise lors dudit mouvement axial et de la rotation relative au plongeur (16) et au cylindre (8) qui en résulte.

2. Seringue selon la revendication 1, dans laquelle l'adaptateur (102) comprend un embout (100) pour la fixation amovible de l'aiguille (2) sur l'adaptateur (102).

3. Seringue selon la revendication 2, dans laquelle l'embout (100) et l'adaptateur (102) comprennent chacun une partie filetée pour l'assemblage de l'un et de l'autre.

4. Seringue selon l'une quelconque des revendications 1 à 3, dans laquelle l'adaptateur (102) est fixé de façon amovible à l'extrémité distale du cylindre (8) par une liaison filetée dans un premier sens, et dans laquelle lesdites surfaces de came (118) comportent une liaison filetée dans un second sens, opposée audit premier sens.

5. Seringue selon l'une quelconque des revendications 1 à 4, comprenant des moyens par lesquels l'aiguille est amenée à entrer en prise amovible avec des moyens d'assemblage de l'aiguille sur l'adaptateur, en réponse à la rotation de l'extrémité du plongeur.

6. Seringue selon l'une quelconque des revendications 1 à 5, dans laquelle l'adaptateur (102) est assemblé de façon amovible à l'extrémité du cylindre (8) par une combinaison de filetage femelle-mâle (110), l'adaptateur (102) comportant un filetage spirale et des surfaces d'entraînement, l'extrémité du plongeur comportant des surfaces d'entraînement pour mettre en prise les surfaces d'entraînement sur l'adaptateur (102) en réponse à la rotation relative de l'extrémité du plongeur (16) et de l'adaptateur (102) et le long dudit filetage, de façon à faire coïncider et à mettre en prise les surfaces d'entraînement de l'extrémité du plongeur et de l'adaptateur (102) respectivement, puis pour faire tourner l'adaptateur (102) par rapport au cylindre (8) de façon à permettre l'extraction axiale de l'adaptateur (102) et de l'embout de l'aiguille le long du cylindre.

7. Seringue selon l'une quelconque des revendications 1 à 6, dans laquelle l'aiguille comporte un raccord de verrouillage Luer femelle, qui est en prise avec l'adaptateur (102) comportant un embout Luer simple ou un raccord de verrouillage Luer mâle.

8. Seringue selon l'une quelconque des revendications 1 à 7, dans laquelle l'adaptateur (102) est assemblé de façon amovible à l'extrémité distale du cylindre (8) par une liaison filetée dans un premier sens, tandis que les structures de mise en prise entre l'adaptateur (102) et le plongeur (16) comprennent une liaison filetée dans le même sens que ledit premier sens.

9. Seringue selon l'une quelconque des revendications 1 à 8, dans laquelle lesdites surfaces de came comprennent une saillie centrale creuse (104) sur le plongeur (16), présentant au moins une piste hélicoïdale (118) autour d'une surface externe de la saillie.

10. Seringue selon la revendication 9, dans laquelle le verrou comprend au moins un moyen d'encliquetage (106), également disposé sur la surface externe de ladite saillie, mais à distance périphérique de laditepiste hélicoïdale.

11. Seringue selon la revendication 10, comprenant une paire desdits moyens d'encliquetage (106), chacun des éléments de ladite paire étant à une distance périphérique environ 180°.

12. Seringue selon l'une quelconque des revendications 1 à 11, dans laquelle lesdits moyens de verrouillage comprennent une encoche sur l'un parmi ledit adaptateur (102) et ledit plongeur (16) et une saillie sur l'autre parmi ledit adaptateur (102) et ledit plongeur (16), destinée à être reçue dans ladite encoche.

13. Seringue selon l'une quelconque des revendications 1 à 12, comprenant un méplat qui s'étend axialement et radialement sur chacun dudit adaptateur (102) et dudit plongeur, ce méplat étant destiné à servir de surfaces d'entraînement au cours de ladite rotation du plongeur.

14. Seringue selon la revendication 13, dans laquelle lesdites surfaces d'entraînement ont une étendue axiale plus grande que leur étendue radiale.

15. Seringue selon l'une quelconque des revendications 1 à 14, comprenant un embout pour porter ladite aiguille, cet embout possédant un filetage mâle, ledit adaptateur (102) comprenant une partie filetée femelle.

16. Seringue selon l'une quelconque des revendications 1 à 15, comprenant des filetages de vis mâle portés par l'extrémité distale du cylindre (8).

17. Seringue selon la revendication 16, comprenant des filetages femelles portés par l'extrémité proximale du plongeur (16), ledit plongeur (16) présentant une faible section intermédiaire entre ses extrémités, afin qu'une partie du plongeur (16) comprenant l'extrémité proximale se détache lors de l'extraction du plongeur (16) de l'extrémité distale du cylindre (8), d'où il résulte que la partie qui est détachée peut être vissée sur le cylindre (8) en permettant aux filetages mâle et femelle de coopérer sur le cylindre (8) et l'extrémité du plongeur respectivement.

18. Seringue selon l'une quelconque des revendications 1 à 17, comprenant, sur le cylindre (8) et le plongeur (16), des moyens destinés à empêcher l'extraction totale du plongeur (16) hors du cylindre (8).

19. Seringue selon l'une quelconque des revendications 1 à 18, dans laquelle les moyens de verrouillage sont tels qu'ils permettent su plongeur et à l'adaptateur d'être libérés si on le désire.
